# EUROPÄISCHE PATENTSCHRIFT

(11) **EP 0 567 953 B1**
(45) Veröffentlichungstag und Bekanntmachung des Hinweises auf die Patenterteilung: **13.03.1996**
(21) Anmeldenummer: 93106648.4
(22) Anmeldetag: 23.04.1993
(51) Int. Cl.: C07C 45/46, C07C 49/67

(54) **Verfahren zur Herstellung substituierter Indanone**
Process for the preparation of substituted indanones
Procédé pour la préparation d'indanones substituées

(30) Priorität: 28.04.1992 DE 4213939
(43) Veröffentlichungstag der Anmeldung: 03.11.1993
(73) Patentinhaber: HOECHST AKTIENGESELLSCHAFT, D-65926 Frankfurt am Main (DE)
(72) Erfinder: Weisse, Laurent, Dr., W-6370 Oberursel (DE); Strutz, Heinz, Dr., W-6390 Usingen (DE)

(56) Entgegenhaltungen:
- EP-A- 0 203 276
- US-A- 1 754 031
- DATABASE WPI Week 7630, Derwent Publications Ltd., London, GB; AN 76-56618X & JP-A-51 065 742
- JOURNAL OF THE AMERICAN CHEMICAL SOCIETY. Bd. 72, 1950, GASTON, PA US Seiten 3286 - 3287 R.T. HART ET AL. 'Acylation - Alkylation Studies. I.'
- JOURNAL OF THE AMERICAN CHEMICAL SOCIETY. Bd. 61, 1939, GASTON, PA US Seiten 1272-1281 L.F. Fieser et al.I 'Inter- and Intramolecular Acylations with Hydrogen Fluoride.'

## Beschreibung

Die vorliegende Erfindung betrifft ein technisch einfaches Verfahren zur Herstellung von substituierten 1-Indanonen.

Verbindungen dieses Typs sind wichtige Zwischenprodukte bei der Herstellung von Metallocen-Komplexen, da sich 1-Indanone leicht in die entsprechenden Indene überführen lassen. Indene werden als Ligandsystem zum Aufbau von Metallocen-Komplexen verwendet (EP-A 336 128). Insbesondere die entsprechenden verbrückten, chiralen Zirkon-Derivate besitzen als hochaktive Katalysatoren bei der Olefinpolymerisation große Bedeutung (vgl. EP-A 129 368; EP-A 321 852). Durch Variation des Ligandsystems, z.B. durch Substitution, können die Katalysatoreigenschatten gezielt beeinflußt werden. Hierdurch ist es möglich, die Polymerausbeute, die Molmasse, die Taktizität oder den Schmelzpunkt der Polymere im gewünschten Maß zu verändern (New J. Chem. 14 (1990) 499; Organomet. 9 (1990) 3098; Angew. Chem. 102 (1990) 339; EP-A 316 155; EP-A 351 392).

Weiterhin besitzen substituierte 1-Indanone technische Bedeutung als Riechstoffe (EP-A 162 465) und als wertvolle Zwischenprodukte bei der Herstellung von pharmazeutischen Produkten oder anderen bioaktiven Verbindungen (EP-A 421 759; J. Med. Chem. 25 (1990) 765).

In der Literatur sind mehrere Verfahren zur Herstellung von substituierten 1-Indanonen beschrieben.

1-Indanone, die am Sechsring Substituenten tragen, können ausgehend von den entsprechend substituierten Aromaten durch Ankondensieren des Fünfrings in 2- bis 6-stufigen Synthesen hergestellt werden (J. Org. Chem., 55 (1990) 247; Bull. Soc. Chim. Fr. 6 (1969) 1981).

Herstellungsverfahren für 1-Indanone, die am Fünfring oder an beiden Ringen Substituenten tragen sind ebenfalls bekannt (J. Org. Chem. 46 (1981) 3758; J. Org. Chem. 23 (1958) 1441).

Diese Methoden haben den Nachteil, daß sie in der Regel mehrstufig sind und nur schlechte Gesamtausbeuten an den gewünschten Produkten liefern. Viele der Synthesen sind nicht allgemein anwendbar, sondern auf spezielle Derivate beschränkt. Bei anderen sind wiederum die Ausgangsmaterialien schlecht zugänglich oder sehr teuer. Gewisse Substitutionsmuster am Aromaten sind ebenfalls nach diesen Methoden nicht realisierbar. Die wenigen bekannten einstufigen Synthesen haben den Nachteil, daß sie auf spezielle Derivate beschränkt sind und schlechte Ausbeuten liefern, so daß technisch aufwendige Reinigungsoperationen der Produkte erforderlich sind. Die meisten dieser Reaktionen werden mit Hilfe von Friedel-Grafts-Katalysatoren, wie z.B. AlCl₃, durchgeführt, die im Überschuß eingesetzt werden. Diese Reaktionen erfordern technisch aufwendige Aufarbeitungsschritte, die mit einem großen Salzanfall verbunden sind.

Bekannt sind auch Herstellungsverfahren von substituierten Indanonen durch Umsetzung von Aromaten wie Xylol oder Acenaphthen mit wäßriger Methacrylsäure, Crotonsäure oder Zimtsäure in einem großen Überschuß von flüssigem Fluorwasserstoff (J. Am. Chem. Soc. 61 (1939) 1272; J. Am. Chem. Soc. 72 (1950) 3287). Die Ausbeuten liegen zwischen 62 % und 81 %. Diese Methode hat den Nachteil, daß durch vorhandenes oder gebildetes Wasser erhebliche Korrosionsprobleme verursacht werden. Eine Recyclisierung des Fluorwasserstoffs ist durch die Anwesenheit von Wasser ebenfalls nicht möglich. Die Flußsäure muß neutralisiert werden, wodurch eine große Menge an schwer entsorgbarem Salz anfällt. Außerdem müssen die Produkte aufgrund der niedrigen Ausbeuten noch gereinigt werden.

Es bestand somit die Aufgabe, ein Verfahren zur Herstellung der obengenannten Indanone zu finden, das die aus dem Stand der Technik bekannten Nachteile vermeidet.

Völlig überraschend wurde gefunden, daß Aromaten der nachstehenden Formel I mit käuflichen Carbonsäureester der Formel II in flüssigem Fluorwasserstoff nahezu quantitativ zu Indanonen der Formel III/IIIa reagieren. Eine aufwendige Reinigung der Produkte ist daher nicht nötig. Außerdem ist dieses Verfahren ein technisch einfach zu handhabender Einstufenprozeß. Da unter den Reaktionsbedingungen die gebildeten Alkohole nicht dehydratisieren, wird die einem technischem Verfahren prohibitive Flußsäure nicht gebildet. Somit stellt diese Methode ein ökonomisch günstiges und neues Herstellungsverfahren für substituierte 1-Indanone dar. Gleichzeitig ermöglicht das Verfahren die Herstellung von neuen Verbindungen des genannten Strukturtyps.

Die vorliegende Erfindung betrifft daher ein Verfahren zur Herstellung einer Verbindung der Formel III oder deren Isomer der Formel IIIa 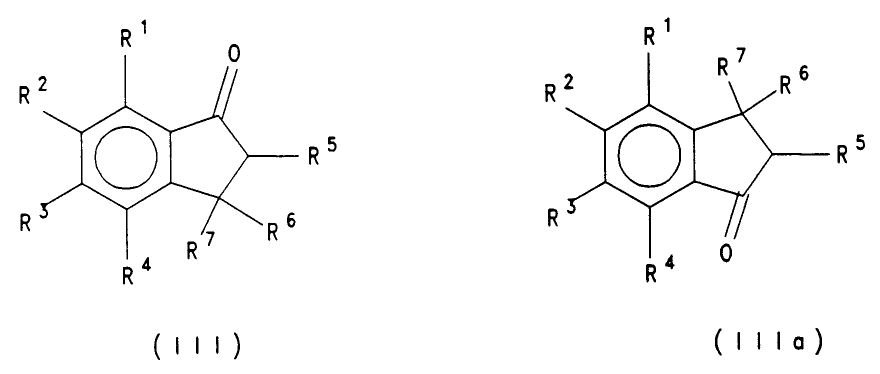
worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest
-SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ mit den sie verbindenden Atomen einen oder mehrere substituierte oder unsubstituierte Ringe bilden, dadurch gekennzeichnet, daß man eine Verbindung der Formel I 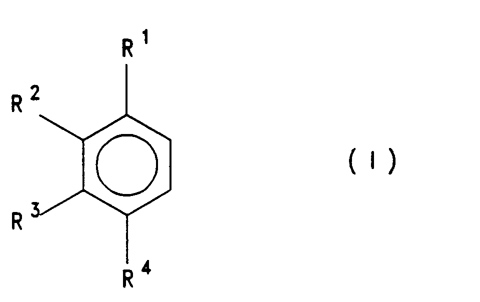
mit einer Verbindung der Formel II 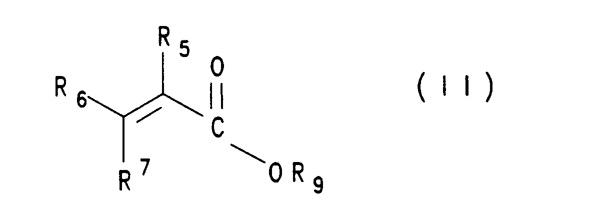
worin R⁹ für (C₁-C₂₀) geradkettiges Alkyl steht und die Substituenten R¹ - R⁷ die genannten Bedeutungen besitzen, in flüssigem, wasserfreiem Fluorwasserstoff umsetzt.

Dabei steht Alkyl für geradkettiges oder verzweigtes Alkyl. Halogen bedeutet Fluor, Chlor, Brom oder Jod, insbesondere Fluor oder Chlor. Beispiele für heteroaromatische Reste sind Thienyl, Furyl oder Pyridyl.

Die von benachbarten Resten R¹-R⁴ gebildeten Ringe können durch Substituenten in der Bedeutung von R¹-R⁷, einschließlich der dafür genannten Vorzugsbereiche, substituiert sein.

In den Formeln III bzw. IIIa gilt bevorzugt, daß R¹, R², R³, R⁴ gleich oder verschieden sind und Wasserstoff, (C₁-C₁₀)Alkyl, (C₁-C₄)Alkoxy, (C₂-C₆)Alkenyl, (C₁-C₆)Fluoralkyl oder ein Halogenatom bedeuten oder die Reste R¹ und R², R² und R³ oder R³ und R⁴ mit den sie verbindenden Atomen einen substituierten oder unsubstituierten fünf- oder sechsgliedrigen Ring bilden, R⁵, R⁶ und R⁷ Wasserstoff und (C₁-C₁₀)Alkyl bedeuten.

Insbesondere gilt, daß R¹, R², R³ und R⁴ gleich oder verschieden sind und Wasserstoff oder (C₁-C₁₀)Alkyl bedeuten, oder die Reste R¹ und R², R² und R³ oder R³ und R⁴ mit den sie verbindenden Atomen einen substituierten oder unsubstituierten sechsgliedrigen oder fünfgliedrigen, gesättigten oder ungesättigten Carbocyclus bilden und R⁵, R⁶ und R⁷ gleich oder verschieden sind und für Wasserstoff oder Methyl stehen.

Der von benachbarten Substituenten R¹ - R⁴ gebildete gesättigte oder ungesättigte Fünf- oder Sechsring (Carbocyclus) kann zusätzlich Substituenten, bevorzugt (C₁-C₁₀)Alkyl, tragen.

Die Indanone können in Abhängigkeit des Substitutionsmusters am Aromaten in Form zweier Konstitutionsisomere der Formel III bzw. IIIa anfallen. Je nach Verwendungszweck können diese in reiner Form oder als Gemisch weiter umgesetzt werden. Bei der Herstellung von Metallocen-Komplexen und bei der Verwendung der 1-Indanone als Reichstoffe kann die Isomerenmischung eingesetzt werden.

Die Ausgangsverbindungen sind im Handel erhältlich oder sie können nach Iiteraturbekannten Methoden hergestellt werden.

Bei der Herstellung der Verbindungen III/IIIa kann dem Fluorwasserstoff zusätzliches Lösemittel zugesetzt werden, bevorzugt wird jedoch in reinem, wasserfreiem Fluorwasserstoff gearbeitet.

Die Molverhältnisse der Ausgangsverbindungen einschließlich des Fluorwasserstoffs können innerhalb weiter Grenzen schwanken. Bevorzugt ist das Molverhältnis von Verbindung I : II : HF = 1 : 0,5-2,0 : 5-100; insbesondere 1 : 0,9-1,2 : 20-50. D.h. es wird in einem Überschuß von Fluorwasserstoff gearbeitet.

Die Reaktionstemperatur beträgt bevorzugt -30°C bis 130°C, insbesondere 0°C bis 80°C.

Die Reaktionszeiten schwanken in der Regel zwischen 30 min und 50 h, vorzugsweise zwischen 1 h und 24 h.

Bevorzugt wird die Reaktion in einem Druckbereich von 1-15 atm durchgeführt.

Bevorzugt wird eine Mischung der Verbindungen I und II vorgelegt und der Fluorwasserstoff zudosiert. Auch die umgekehrte Zugabefolge ist möglich.

Nach Ende der Reaktion kann der Fluorwasserstoff abdestilliert und nahezu quantitativ ohne nennenswerte Verunreinigungen zurückgewonnen werden. Der Rückstand kann dann durch Destillation von dem gebildeten Alkohol oder Spuren Fluorwasserstoff befreit werden.

Die Indanone der Formel III bzw. IIIa können durch Waschen mit Na₂CO₃-, NaHCO₃- oder KOH-Lösung und Wasser von Säureanteilen befreit und mit den üblichen Trockenmitteln wie Na₂SO₄, MgSO₄ oder Molekularsieben getrocknet werden. Da die Umsetzungen in der Regel nahezu quantitativ sind, kann in den meisten Fällen auf eine weitere Reinigung verzichtet werden. Oft empfiehlt sich jedoch eine Filtration über Kieselgel, Aluminiumoxid oder Filterhilfsmitteln, wie z.B. Celite. Falls notwendig, kann die weitere Reinigung durch Destillation, Säulenchromatographie oder Kristallisation erfolgen. Falls erforderlich können die Konstitutionsisomeren III und IIIa durch Säulenchromatographie an Kieselgel oder Aluminiumoxid voneinander getrennt werden.

Das erfindungsgemäße Verfahren zeichnet sich besonders dadurch aus, daß unterschiedlich substituierte 1-Indanone in einer einfachen und kurzen Synthese (Einstufenprozeß) sehr selektiv und in nahezu quantitativer Ausbeute erhalten werden können. Eine aufwendige Reinigung der Derivate ist daher im Gegensatz zum Stand der Technik nicht notwendig. Ein weiterer Vorteil ist, daß der als Katalysator dienende Fluorwasserstoff größtenteils zurückgewonnen und wiederverwendet werden kann, da während der Reaktion kein Wasser gebildet wird. Dies hat den weiteren technisch entscheidenden Vorteil, daß Korrosionsprobleme, verursacht durch wäßrige Flußsäure, vermieden werden. Somit stellt diese Methode ein ökonomisch und ökologisch sehr günstiges Herstellungsverfahren für substituierte 1-Indanone dar. Das Substitutionsmuster am Fünf- und Sechsring kann dabei in einem sehr weiten Bereich variiert werden. Dadurch sind auch neue 1-Indanonderivate zugänglich.

Bevorzugt werden die Indanone III/IIIa zur Herstellung von Metallocenen (vgl. z.B. EP-A 336 128) oder als Riechstoffe (EP-A 162 465) verwendet. Zur Darstellung der Metallocene werden die Indanone, bevorzugt als Isomerengemisch, zunächst nach literaturbekannten Methoden mit Reduktionsmitteln wie NaBH₄ oder LiAlH₄ zu den entsprechenden Indanolen reduziert und diese anschließend mit Säuren wie Schwefelsäure, Oxalsäure, p-Toluolsulfonsäure oder auch durch Behandlung mit wasserentziehenden Substanzen wie Magnesiumsulfat, Natriumsulfat, Aluminiumoxid, Kieselgel oder Molekularsieben zu den entsprechenden Indenen dehydratisiert (Bull. Soc. Chim. Fr. 11 (1973) 3092; Organomet. 9 (1990) 3098).

Die substituierten Indene können als Doppelbindungsisomere anfallen. Diese können von Nebenprodukten durch Destillation, Säulenchromatographie oder Kristallisation gereinigt werden. Die Isomere können als Gemisch direkt für die Synthese der entsprechenden Metallocen-Komplexe eingesetzt werden.

Die Synthese der Metallocene, ausgehend von Indenen, ist bekannt (AU-A-31478/89; J. Organomet. Chem. 342 (1988) 21; EP-A 284 707).

Die nachfolgenden Beispiele dienen der näheren Erläuterung der Erfindung.

### Beispiel A

### 3,3,4,6-Tetramethyl-1-indanon (1) und 3,3,5,7-Tetramethyl-1-indanon (1a)

10,6 g (100 mmol) m-Xylol (99 %) und 14,4 g (112 mmol) 3,3-Dimethylacrylsäureethylester wurden in einem 250 ml Edelstahlautoklaven mit 100 g (5 mol) wasserfreiem Fluorwasserstoff versetzt und 18 h bei 50°C gerührt. Anschließend wurde der Fluorwasserstoff abdestilliert, der Rückstand in Essigester aufgenommen und mit verdünnter KOH-Lösung neutralisiert. Die abgetrennte wäßrige Phase wurde noch zweimal mit Essigester extrahiert. Die vereinigten organischen Phasen wurden mit MgSO₄ getrocknet und unter vermindertem Druck vom Lösemittel befreit. Man erhielt 18,5 g eines hellbraunen Öls. Die Selektivitäten zu (1) bzw. (1a) betrugen 78 % bzw. 21 %. (Ausbeute: 76% bzw. 20,7% d. Th.).
¹H-NMR-Spektren (100 MHz, CDCl₃): 1: 7,36 (d,1H), 7,18 (d,1H), 2,57 (s,2H), 2,47 (s,3H), 2,32 (s,3H), 1,47 (s,6H); 1a: 7,07 (d,1H), 6,9 (d,1H), 2,6-2,3 (m,8H), 1,37 (s,6H)

### Beispiel B

### 3,3,4,7-Tetramethyl-1-indanon (2)

10,6 g (100 mmol) p-Xylol (99 %) und 14,4 g (112 mmol) 3,3-Dimethylacrylsäureethylester wurden analog Beispiel A mit flüssigem Fluorwasserstoff umgesetzt und aufgearbeitet. Man erhielt 18 g der Verbindung (2) in einer Reinheit von 96 % (GC). (Ausbeute: 92 % d.Th.).
¹H-NMR-Spektren (100 MHz, CDCl₃): 2: 7,17 (d,1H), 6,99 (d,1H), 2,57 (s,2H), 2,55 (s,3H), 2,45 (s,3H), 1,47 (s,3H)

### Beispiel C

### 3,3-Dimethyl-1-indanon (3)

7,81 g (100 mmol) Benzol und 14,4 g (112 mmol) 3,3-Dimethylacrylsäureethylester wurden analog mit 100 g (5 mol) flüssigem Fluorwasserstoff umgesetzt und bei 70°C 1 Stunde gerührt. Die Aufarbeitung erfolgte analog Beispiel A. Man erhielt 15,5 g der Verbindung (3) mit einer Reinheit von 96% (GC). (Ausbeute: 93,1% d.Th.). ¹H-NMR-Spektren (100 MHz, CDCl₃): 3: 7,77-7,25 (m,4H), 2,57 (s,2H), 1,37 (s,6H).

### Beispiel D

### 4,7-Dimethyl-3-phenyl-1-indanon (4)

10,6 g (100 mmol) p-Xylol (99 %) und 17 g (105 mmol) trans-Zimtsäuremethylester wurden mit 100 g (5 mol) wasserfreiem Fluorwasserstoff versetzt und 17 Stunden bei 70°C gerührt. Nach der analog Beispiel A erfolgten Aufarbeitung erhielt man 23,6 g eines gelblichen Feststoffs. Die Reinheit des Produktes ist 96 % (GC). (96 % d.Th.). Nach einmaligem Umkristallisieren aus einem Essigester/Hexan-Gemisch (1:1) ließ sich mit 90 % Ausbeute ein nahezu weißer Feststoff gewinnen. Die Reinheit ist nach diesem Schritt größer 98 % (GC).
¹H-NMR-Spektrum (300 MHz, CDCl₃): 4: 7,27-6,97 (m, 7H), 4,48 (dd, 1H), 3,16 (dd, 1H), 2,66 (s, 3H), 2,54 (dd, 1H), 1,95 (s, 3H).

### Beispiel E

### 3,4,7-Trimethyl-1-indanon (5)

10,6 g (100 mmol) p-Xylol und 12,5 g (109 mmol) Crotonsäureethylester wurden mit 100 g (5 mol) Fluorwasserstoff versetzt und 20 h bei 70°C gerührt. Nach der analog Beispiel A erfolgten Aufarbeitung erhielt man 12 g der Verbindung (5) mit einer Reinheit von 80 % (GC) (Ausbeut: 55 % d.Th.).

## Patentansprüche

1. Verfahren zur Herstellung einer Verbindung der Formel III oder deren Isomer der Formel IIIa 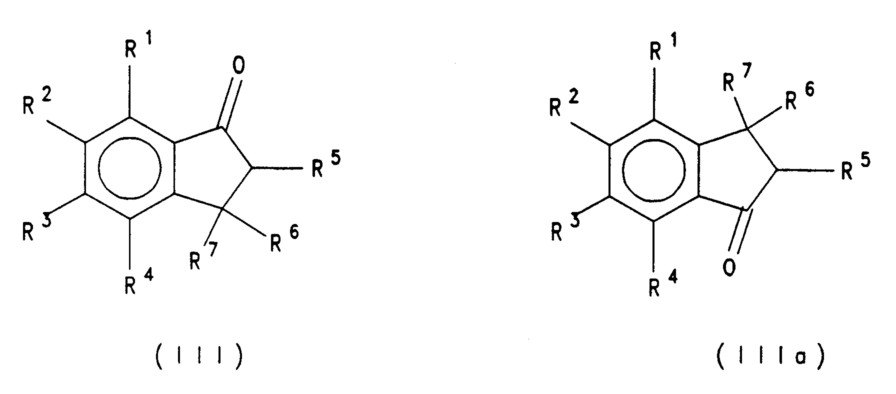 worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest
-SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹, R², R³ und R⁴ mit den sie verbindenden Atomen einen oder mehrere substituierte oder unsubstituierte Ringe bilden, dadurch gekennzeichnet, daß man eine Verbindung der Formel I 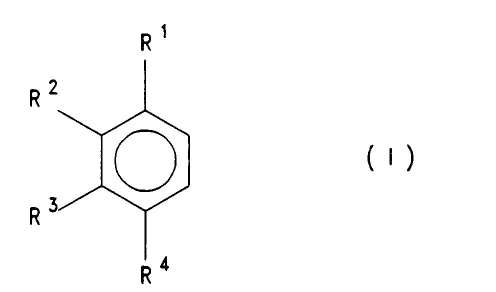 mit einer Verbindung der Formel II 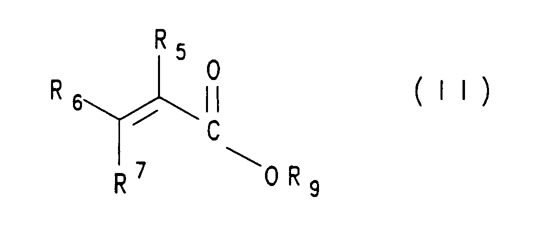 worin R⁹ für (C₁-C₂₀) geradkettiges Alkyl steht und R¹ bis R⁷ die genannten Bedeutungen besitzen, in flüssigem, wasserfreiem Fluorwasserstoff umsetzt.

2. Verfahren gemäß Anspruch 1, dadurch gekennzeichnet, daß in den Formeln III und IIIa R¹, R², R³, R⁴ gleich oder verschieden sind und Wasserstoff, (C₁-C₁₀)Alkyl, (C₁-C₄)Alkoxy, (C₂-C₆)Alkenyl, (C₁-C₆)Fluoralkyl oder ein Halogenatom bedeuten oder die Reste R¹ und R², R² und R³ oder R³ und R⁴ mit den sie verbindenden Atomen einen substituierten oder unsubstituierten fünf- oder sechsgliedrigen Ring bilden, R⁵, R⁶ und R⁷ Wasserstoff oder (C₁-C₁₀)Alkyl bedeuten.

3. Verfahren gemäß Anspruch 1 oder 2, dadurch gekennzeichnet, daß in den Formeln III und IIIa R¹, R², R³ und R⁴ gleich oder verschieden sind und Wasserstoff oder (C₁-C₁₀)Alkyl bedeuten, oder die Reste R¹ und R², R² und R³ oder R³ und R⁴ mit den sie verbindenden Atomen einen substituierten oder unsubstituierten sechsgliedrigen, gesättigten oder ungesättigten Carbocyclus bilden und R⁵, R⁶ und R⁷ gleich oder verschieden sind und für Wasserstoff oder Methyl stehen.

4. Verfahren gemäß einem oder mehreren der Ansprüche 1 bis 3, dadurch gekennzeichnet, daß das molare Verhältnis von Verbindung I: Verbindung II : Fluorwasserstoff 1: 0,5-2,0 : 5-100 beträgt.

## Claims

1. A process for the preparation of a compound of the formula III or the isomer thereof of the formula IIIa 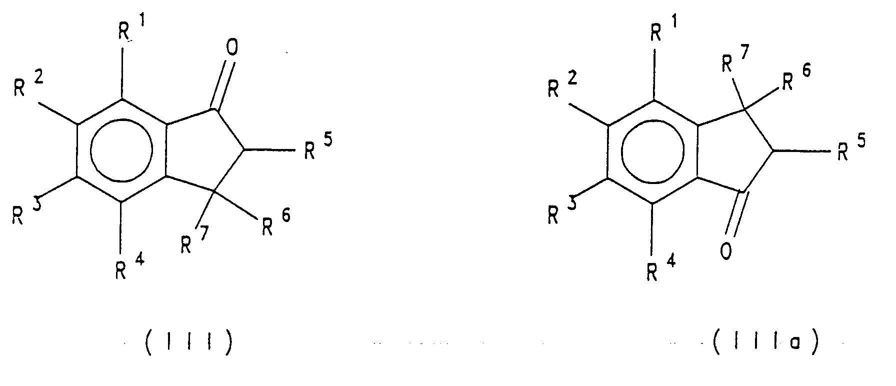 in which
R¹, R², R³, R⁴, R⁵, R⁶ and R⁷ are identical or different and are hydrogen, (C₁-C₂₀)-alkyl, (C₅-C₁₄)aryl, (C₁-C₁₀)alkoxy, (C₂-C₁₀)alkenyl, (C₇-C₂₀)arylalkyl, (C₇-C₂₀)alkylaryl, (C₆-C₁₀)aryloxy, (C₁-C₁₀)fluoroalkyl, (C₆-C₁₀)haloaryl, (C₂-C₁₀)alkynyl, an -SiR⁸₃ radical in which R⁸ is (C₁-C₁₀)alkyl, or are a halogen atom or a heteroaromatic radical having 5 or 6 ring memoers which may contain one or more heteroatoms, or the adjacent radicals R¹, R², R³ and R⁴, together with the atoms connecting them, form one or more substituted or unsubstituted rings, which comprises reacting a compound of the formula I 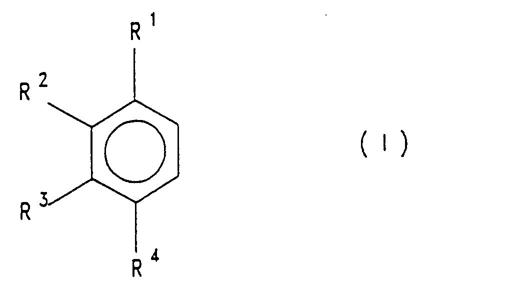 with a compound of the formula II 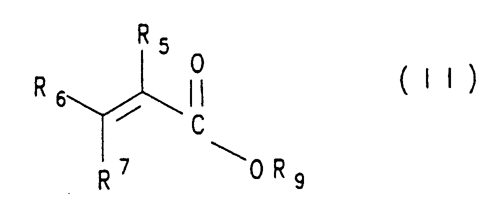 in which R⁹ is straight-chain (C₁-C₂₀)alkyl, and R¹ to R⁷ are as defined above, in liquid, anhydrous hydrogen fluoride.

2. The process as claimed in claim 1, wherein, in the formulae III and IIIa, R¹, R², R³ and R⁴ are identical or different and are hydrogen, (C₁-C₁₀)alkyl, (C₁-C₄)alkoxy, (C₂-C₆)alkenyl, (C₁-C₆)fluoroalkyl or a halogen atom, or the radicals R¹ and R², R² and R³ or R³ and R⁴, together with the atoms connecting them, form a substituted or unsubstituted, five- or six-membered ring, and R⁵, R⁶ and R⁷ are hydrogen or (C₁-C₁₀)alkyl.

3. The process as claimed in claim 1 or 2, wherein, in the formulae III and IIIa, R¹, R², R³ and R⁴ are identical or different and are hydrogen or (C₁-C₁₀)alkyl, or the radicals R¹ and R², R² and R³ or R³ and R⁴, together with the atoms connecting them, form a substituted or unsubstituted, six-membered, saturated or unsaturated carbocyclic ring, and R⁵, and R⁶ and R⁷ are identical or different and are hydrogen or methyl.

4. The process as claimed in one or more of claims 1 to 3, wherein the molar ratio between compound I: compound II : hydrogen fluoride is 1:0.5-2.0:5-100.

## Revendications

1. Procédé de préparation d'un composé de formule III ou de son isomère de formule IIIa 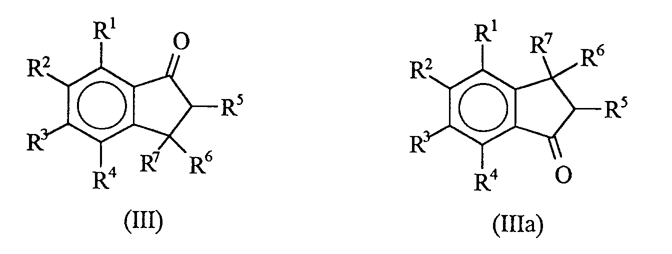 où
R¹, R², R³, R⁴, R⁵, R⁶ et R⁷ sont identiques ou différents et représentent un atome d'hydrogène, un reste alkyle en C₁-C₂₀, aryle en C₆-C₁₄, alcoxy en C₁-C₁₀, alcényle en C₂-C₁₀, arylalkyle en C₇-C₂₀, alkylaryle en C₇-C₂₀, aryloxy en C₆-C₁₀, fluoroalkyle en C₁-C₁₀, halogénoaryle en C₆-C₁₀ ou alcynyle en C₂-C₁₀, un reste -SiR⁸₃ dans lequel R⁸ représente un groupe alkyle en C₁-C₁₀, un atome d'halogène ou un reste hétéroaromatique de 5 ou 6 chaînons qui peut contenir un ou plusieurs hétéroatomes, ou des restes R¹, R², R³ et R⁴ adjacents forment avec les atomes qui les relient un ou plusieurs cycles substitués ou non substitués, caractérisé en ce que l'on fait réagir dans de l'acide fluorhydrique liquide anhydre un composé de formule I 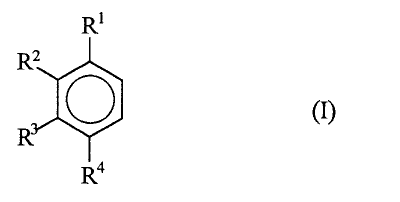 avec un composé de formule II 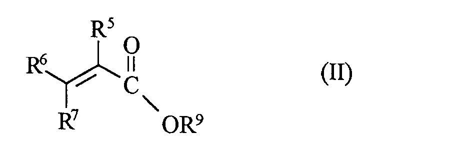 où R⁹ représente un reste alkyle linéaire en C₁-C₂₀ et les R¹ à R⁷ ont les significations indiquées.

2. Procédé selon la revendication 1, caractérisé en ce que, dans les formules III et IIIa, R¹, R², R³, R⁴ sont identiques ou différents et représentent un atome d'hydrogène, un reste alkyle en C₁-C₁₀, alcoxy en C₁-C₄, alcényle en C₂-C₆ ou fluoroalkyle en C₁-C₆ ou un atome d'halogène, ou les restes R¹ et R², R² et R³ ou R³ et R⁴ forment avec les atomes qui les relient un cycle à 5 ou 6 chaînons substitué ou non substitué, et R⁵, R⁶ et R⁷ représentent l'hydrogène ou des restes alkyle en C₁-C₁₀.

3. Procédé selon la revendication 1 ou 2, caractérisé en ce que, dans les formules III et IIIa, R¹, R², R³ et R⁴ sont identiques ou différents et représentent des atomes d'hydrogène ou des restes alkyle en C₁-C₁₀, ou les restes R¹ et R², R² et R³, ou R³ et R⁴ forment avec les atomes qui les relient un carbocycle à 5 ou 6 chaînons substitué ou non substitué, saturé ou insaturé, et R⁵, R⁶ et R⁷ sont identiques ou différents et représentent des atomes d'hydrogène ou des restes méthyle.

4. Procédé selon l'une ou plusieurs des revendications 1 à 3, caractérisé en ce que le rapport molaire composé I : composé 2 : acide fluorhydrique est de 1 : 0,5-2,0 : 5-100.
